# FASCICULE DE BREVET EUROPEEN

(11) **EP 3 047 255 B1**
(45) Date de publication et mention de la délivrance du brevet: **06.11.2019**
(21) Numéro de dépôt: 14790194.6
(22) Date de dépôt: 18.09.2014
(51) Int. Cl.: G01N 21/03, G01N 21/3504, G01N 21/01, G01N 21/05, G01N 33/497, G01N 21/15

(54) **APPAREIL DE MESURE PAR SPECTROMÉTRIE COMPRENANT UNE CELLULE DE MESURE COMBINÉE À UN DISPOSITIF DE CHAUFFAGE**
SPEKTROMETRIEMESSVORRICHTUNG MIT EINER MESSZELLE IN KOMBINATION MIT EINER ERWÄRMUNGSVORRICHTUNG
SPECTROMETRY MEASUREMENT APPARATUS COMPRISING A MEASURING CELL COMBINED WITH A HEATING DEVICE

(30) Priorité: 18.09.2013 FR 1358964
(43) Date de publication de la demande: 27.07.2016
(73) Titulaire: Olythe, 13100 Aix-en-Provence (FR)
(72) Inventeur: NESA, Guillaume, F-13090 Aix en Provence (FR); DUBOIS, Grégory, 78600 Maisons-Laffitte (FR); FLESCH, Etienne, F-78570 Andresy (FR); PAFUMI, Lionel, F-13127 Vitrolles (FR); THIERRY, Jean-Pierre, F-78570 Andresy (FR)
(74) Mandataire: Roman, Alexis
(86) Numéro de dépôt international: PCT/FR2014/052319
(87) Numéro de publication internationale: WO 2015/040329

(56) Documents cités:
- WO-A1-02/096290
- WO-A1-2011/042679
- FR-A1- 2 941 530
- JP-A- 2012 177 690
- US-A1- 2011 084 820
- DATABASE WPI Week 200954 Thomson Scientific, London, GB; AN 2009-M36899 XP002724903, -& CN 201 282 572 Y (LIANG J) 29 juillet 2009 (2009-07-29) cité dans la demande

## Description

### Domaine technique de l'invention.

L'invention a pour objet un appareil de mesure par spectrométrie comprenant une cuve de mesure combinée à un dispositif de chauffage. Elle a encore pour objet un éthylomètre pour mesurer ou détecter un taux de gaz partiel exhalé par un fluide d'haleine et comprenant un tel appareil. Elle a enfin pour objet un procédé de fabrication d'une cuve de mesure d'un éthylomètre dans laquelle est inséré ce dispositif.

L'invention concerne le domaine technique des éléments constitutifs des appareils de mesure par spectrométrie, et plus particulièrement des éléments intégrés dans leur cellule de mesure. Elle concerne également le domaine technique des dispositifs électroniques portables, tels que les éthylomètres par exemple, pour mesurer ou détecter un taux de gaz partiel exhalé par un fluide d'haleine.

### État de la technique.

On connait par le document brevet FR 2.941.530 (SERES ENVIRONNEMENT), ci-après « document SERES », un éthylomètre comportant un dispositif d'émission d'un rayonnement infrarouge, un récepteur infrarouge et une cuve de mesure dans laquelle circule le fluide d'haleine dont un taux de gaz partiel doit être mesuré ou détecté. La cuve de mesure se présente sous la forme d'un tube creux dont la surface interne est pourvue d'un matériau réfléchissant formant une couche de réflexion optique. Ce document enseigne d'associer le tube creux à un moyen de chauffage. Ce dernier permet de porter le tube à une température d'environ 39°C pour éviter toute formation de condensation sur sa paroi interne lors de la circulation du fluide d'haleine. En effet, une condensation à l'intérieur du tube altère la qualité réfléchissante de la couche de réflexion et ne permet pas une récupération optimale, au niveau du récepteur infrarouge, du rayonnement émis par le dispositif d'émission.

Le document SERES prévoit d'enrouler une résistance chauffante sur la surface externe du tube. Si cette disposition permet un chauffage du tube, il y a néanmoins une grande déperdition thermique. Il en résulte que la consommation électrique n'est pas négligeable, surtout dans le cas d'un appareil portable où la plus grande autonomie énergétique est souhaitée. En outre, la mise en place de la résistance chauffante autour du tube n'est pas aisée et le contact résistance/tube n'est pas assuré. Il en résulte une température de chauffage non homogène sur la surface interne du tube, cette disparité pouvant influencer le trajet optique et donc la mesure finale.

L'invention vise à remédier à cet état des choses. En particulier, un objectif de l'invention est de proposer un dispositif de chauffage qui soit plus économique en termes de consommation électrique, tout en gardant la même qualité optique.

Un autre objectif de l'invention est de proposer un dispositif de chauffage dont la mise en place est plus simple et plus rapide que celui décrit dans le document SERES.

Encore un autre objectif de l'invention est de proposer un dispositif de chauffage qui puisse chauffer de manière plus rapide et homogène le tube creux de la cuve de mesure.

Un objectif supplémentaire de l'invention est de proposer un dispositif de chauffage qui soit adaptable à tout type d'appareil de mesure par spectrométrie.

### Divulgation de l'invention.

La solution proposée par l'invention est un appareil de mesure par spectrométrie comprenant une cuve de mesure combinée à un dispositif de chauffage, ladite cuve de mesure se présentant sous la forme d'un tube creux. Cet appareil remarquable en ce que le dispositif de chauffage se présente sous la forme d'un article optique souple comprenant un support flexible souple présentant une face supérieure et une face inférieure. La face supérieure est recouverte d'un matériau réfléchissant pour former une couche de réflexion optique. Un élément chauffant flexible est disposé sur au moins une des faces du support. Le dispositif de chauffage est inséré dans le tube creux de manière à ce que le matériau réfléchissant de l'article optique souple forme la couche de réflexion optique.

On peut ainsi facilement et rapidement insérer cet article optique dans le tube creux de la cuve de mesure de manière à ce que le matériau réfléchissant dudit article forme la couche de réflexion optique. L'élément chauffant flexible forme le moyen de chauffage et remplace la résistance électrique chauffante prévue dans le document SERES. Cet article optique souple a donc une double fonction : d'une part, assurer la réflexion optique et d'autre part, chauffer la surface réfléchissante. L'élément chauffant étant maintenant disposé à l'intérieur même du tube creux, la déperdition thermique est réduite, voire négligeable, ce qui est bénéfique en terme de réduction de la consommation électrique. En outre, il a été constaté que la montée en température à l'intérieur du tube creux était plus rapide qu'avec le dispositif chauffant du document SERES, et que la répartition de la température était plus homogène.

Le document brevet CN 201.282.572 (JIANBO LIANG) concerne un film chauffant souple avec une face de type radiant. Ce film peut être utilisé dans un grand nombre de domaines, comme l'hygiène quotidienne, la santé, ainsi que pour la conservation de chaleur, la protection contre le gel ou l'humidité pour l'élevage agricole, l'électroménager personnel et les canalisations, et peut-être employé en une variété de lieux dans lesquels la température est basse, moyenne ou haute. Ce film chauffant n'est toutefois pas adapté pour être utilisé pour un appareil de mesure par spectrométrie. En tout état de cause, la couche réfléchissante décrite dans ce document brevet, a pour unique fonction de réfléchir le rayonnement thermique émis pour le filament chauffant, et ce afin d'éviter les déperditions thermiques. En aucun cas, cette couche n'est utilisée pour assurer une réflexion optique.

Le document brevet WO 2011/042679 (AUTOLIV) ne divulgue pas de support flexible souple.

D'autres caractéristiques avantageuses de l'appareil objet de l'invention sont listées ci-dessous, chacune de ces caractéristiques pouvant être considérée seule ou en combinaison avec les caractéristiques remarquables définies ci-dessus :
- L'élément chauffant peut être disposé sur la face inférieure du support flexible.
- L'élément chauffant peut également être disposé sur la face supérieure du support flexible, le matériau réfléchissant recouvrant ledit élément chauffant.
- L'élément chauffant se présente préférentiellement sous la forme d'un circuit imprimé flexible installé sur une face du support, et dans lequel est intégré un ou plusieurs filaments chauffants.
- Le circuit imprimé flexible est préférentiellement associé à une couche d'un matériau conducteur de chaleur.
- Le matériau conducteur de chaleur peut être préférentiellement choisi dans le groupe suivant : cuivre, aluminium, argent, or, etc.
- Le support flexible souple est avantageusement réalisé dans un matériau choisi dans le groupe suivant : polyimide, polyépoxyde, polyester, résine époxy renforcée de fibre de verre.
- Ce support flexible souple a préférentiellement une épaisseur comprise entre 1 µm et 250 µm.
- Le matériau réfléchissant formant la couche de réflexion optique, est avantageusement choisi dans le groupe suivant : or, cobalt, argent, nickel, cuivre, aluminium, chrome, zinc, silice.

Encore un autre aspect de l'invention concerne un éthylomètre pour mesurer ou détecter un taux de gaz partiel exhalé par un fluide d'haleine comprenant un appareil de mesure par spectrométrie selon la revendication 1, ledit éthylomètre comportant un dispositif d'émission d'un rayonnement infrarouge, un récepteur infrarouge et la cuve de mesure dans laquelle peut circuler le fluide d'haleine.

D'autres caractéristiques avantageuses de l'éthylomètre objet de l'invention sont listées ci-dessous, chacune de ces caractéristiques pouvant être considérée seule ou en combinaison avec les caractéristiques remarquables définies ci-dessus :
- Le tube creux formant une cuve de mesure a préférentiellement une longueur inférieure ou égale à 100 mm, l'article optique ayant une longueur correspondante à celle dudit tube.
- La surface interne du tube creux comporte des éléments en relief, l'article optique n'étant en contact avec la surface interne dudit tube qu'au niveau de ces éléments en relief.

Un aspect supplémentaire de l'invention concerne un procédé de fabrication d'une cuve de mesure d'un éthylomètre permettant de mesurer le taux de gaz partiel exhalé par un fluide d'haleine, laquelle cuve de mesure se présente sous la forme d'un tube creux. Ce procédé consiste à :
- utiliser un dispositif de chauffage se présentant sous la forme d'un article optique souple, lequel article comprend un support flexible souple présentant une face supérieure et une face inférieure, la face supérieure étant recouverte d'un matériau réfléchissant pour former une couche de réflexion optique, un élément chauffant flexible étant disposé sur au moins une desdites faces du support,
- rouler ou plier l'article optique du dispositif de chauffage défini précédemment,
- insérer l'article optique ainsi roulé ou plié dans le tube creux de manière à ce que le matériau réfléchissant dudit article forme la couche de réflexion optique.

### Description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
- la figure 1 est une vue schématique en coupe d'un article optique conforme à l'invention,
- la figure 2 est une vue schématique en coupe d'un article optique conforme à l'invention, dans une variante de réalisation,
- la figure 3 est une vue schématique en coupe longitudinale, d'un éthylomètre conforme à l'invention,
- la figure 4 est une vue en coupe selon A-A de l'éthylomètre de la figure 3,
- la figure 5 est une vue schématique de dessus d'un article optique selon la figure 1 ou 2.

### Modes préférés de réalisation de l'invention.

Le dispositif de chauffage, objet de l'invention est particulièrement, mais non exclusivement, destiné à être utilisé dans un appareil de mesure par spectrométrie. Il est notamment conçu pour être combiné à une cellule de mesure d'un tel appareil. Il est notamment conçu pour être intégré dans un éthylomètre, mais peut également être intégré dans tout autre appareil qui mesure un paramètre (concentration d'alcool, de CO, de CO2, de H₂O, ...), dans un fluide d'haleine ou dans tout autre fluide plus chaud et/ou plus humide que l'air ambiant (par exemple une vapeur ou un gaz d'échappement). Sa fonction principale est de maintenir le fluide à analyser à une température souhaitée. Pour une mesure de fluide d'haleine, cette température de chauffe, comprise entre 35°C et 40°C, est telle que le fluide ne condense pas.

Dans un souci de clarté et de concision, la suite de la description fait uniquement référence à un éthylomètre, sans que cela puisse être considéré comme une limitation de la protection recherchée. Par « éthylomètre », on entend au sens de la présente invention tout appareil (y compris les éthylotests) permettant de mesurer ou détecter un taux de gaz partiel exhalé par un fluide d'haleine, et en particulier de mesurer la concentration d'alcool dans l'air expiré et/ou de détecter un seuil de concentration d'alcool dans l'air expiré.

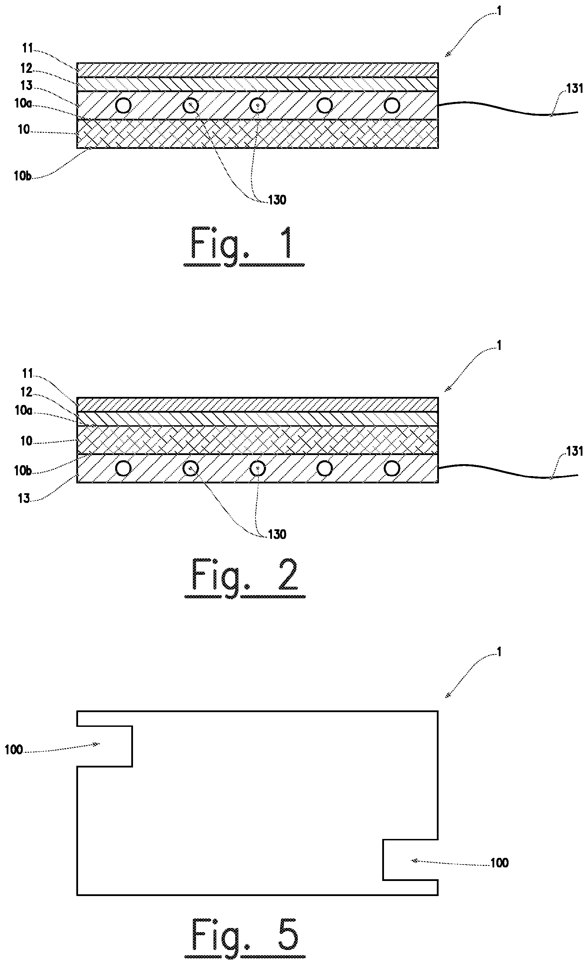

En se rapportant à la figure 1, le dispositif de chauffage se présente sous la forme d'un article optique souple 1. Cet article comprend un support flexible souple 10 qui consiste en un film mince ayant une épaisseur comprise entre 1 µm et 250 µm, préférentiellement environ 25 µm. Un bon rapport souplesse/résistance est obtenu avec ces valeurs d'épaisseur. Sa longueur et sa largeur dépendent des dimensions de la cellule de mesure à laquelle le dispositif de chauffage est combiné. Le support 10 est avantageusement réalisé dans un matériau choisi dans le groupe suivant : polyimide (par ex : Kapton®), polyépoxyde, polyester, résine époxy renforcée de fibre de verre, substrat d'aluminium (par ex : support COOL-CLAD® commercialisé par la société AI TECHNOLOGY). Tout autre matériau généralement utilisé pour la fabrication de circuit imprimé souple peut toutefois être envisagé. Le support 10 peut être obtenu par moulage, extrusion, laminage, etc.

Le support 10 comprend une face supérieure 10a et une face inférieure 10b qui sont opposées. Sur les figures annexées, la face supérieure 10a est recouverte d'un matériau réfléchissant 11 pour former une couche de réflexion optique sur laquelle rebondira le rayonnement infrarouge. Pour que la couche de réflexion soit la plus réfléchissante possible et afin de limiter les pertes énergétiques du rayonnement émis, le matériau réfléchissant 11 est préférentiellement choisi dans le groupe suivant : or, cobalt, argent, nickel, cuivre, aluminium, chrome, zinc, silice.

Le matériau réfléchissant 11 présente une épaisseur comprise entre 0,01 µm et 500 µm. Il peut être déposé par collage, par dépôt électrochimique, par dépôt électrolytique, par impression, par sérigraphie, par métallisation sous vide, chauffage, ou par tout autre procédé d'adhésion de couche fine.

Dans le but d'assurer un bon maintien en position du matériau réfléchissant 11 sur la face supérieure 10a du support 10, une couche d'accroche 12 peut préalablement être déposée sur cette face. Cette couche 12 est par exemple constituée d'une couche de cuivre, d'aluminium, d'argent, ou de polyéthylène, dont l'épaisseur est par exemple comprise entre 0,1 µm et 500 µm, déposée par un procédé d'adhésion de couche fine du type mentionné au paragraphe précédent. La couche 12 n'est pas indispensable et peut notamment être évitée dans le cas où le matériau réfléchissant 11 est par exemple déposé par dépôt électrolytique.

Un élément chauffant 13 flexible est disposé sur au moins une des faces 10a, 10b du support 10. Cet élément chauffant 13 peut consister en une résistance chauffante mince fixée sur le support 10, par exemple par collage, par laminage, par dépôt électrochimique, par dépôt électrolytique, par impression, par sérigraphie, par métallisation sous vide, par chauffage, par fixation mécanique, ou par tout autre procédé d'adhésion de couche fine.

Selon une caractéristique avantageuse de l'invention, l'élément chauffant 13 se présente sous la forme d'un circuit imprimé flexible dans lequel sont intégrés un ou plusieurs filaments chauffants 130. Ces derniers se présentent par exemple sous la forme de bandes de métal (cuivre, cuivre-nickel, aluminium, ...), d'une épaisseur de 1 µm à 50 µm, placées entre deux couches de polyimide. Une de ces couches peut d'ailleurs former le support 10. A titre d'exemple illustratif, on peut utiliser un circuit chauffant souple commercialisé par la société MINCO, sous la marque THERMOFOIL®.

L'élément chauffant 13 est relié à des fils conducteurs 130 qui sortent du support 10 et qui sont destinés à être connectés à une source de courant du type batterie à piles. En pratique, la source de courant est déterminée pour délivrer une tension comprise entre 0,1 Volts et 5 Volts de sorte que la puissance développée par l'élément chauffant 13 soit comprise entre 10 mW/cm² et 2 W/cm². Une régulation en température de l'élément chauffant 13, par exemple autour de 39°C, peut être prévue.

Dans le but d'homogénéiser la répartition de la température sur toute la surface de l'article 1, l'élément chauffant 13 peut être associé à une couche d'un matériau conducteur de chaleur. Sur les figures 1 et 2 annexées, et dans le but de simplifier la conception et d'optimiser la compacité de l'article 1, cette couche est la couche d'accroche 12 précitée, choisie dans un matériau conducteur de chaleur (cuivre, d'aluminium, d'argent, ...). Une autre couche additionnelle peut toutefois être envisagée, en particulier dans le cas où la couche d'accroche n'est pas prévue.

Sur la figure 1, l'élément chauffant 13 est disposé sur la face supérieure 10a du support 10. Il est recouvert par le matériau réfléchissant 11, et éventuellement par la couche 12.

Sur la figure 2, l'élément chauffant 13 est disposé sur la face inférieure 10b du support 10. Il est donc situé à l'opposé du matériau réfléchissant 11. Dans cette configuration, le support 10 joue un rôle de tampon thermique. En effet, comme cela est expliqué plus après, le fluide à analyser circule dans la cuve de mesure en étant au contact du matériau réfléchissant 11, lequel est susceptible de refroidir brusquement. Il a été constaté que dans la configuration de la figure 2, le matériau réfléchissant 11 refroidissait plus lentement, le support 10 augmentant l'inertie thermique de l'article 1. La régulation en température est ainsi plus aisée et moins brusque que dans le cas de la configuration de la figure 1.

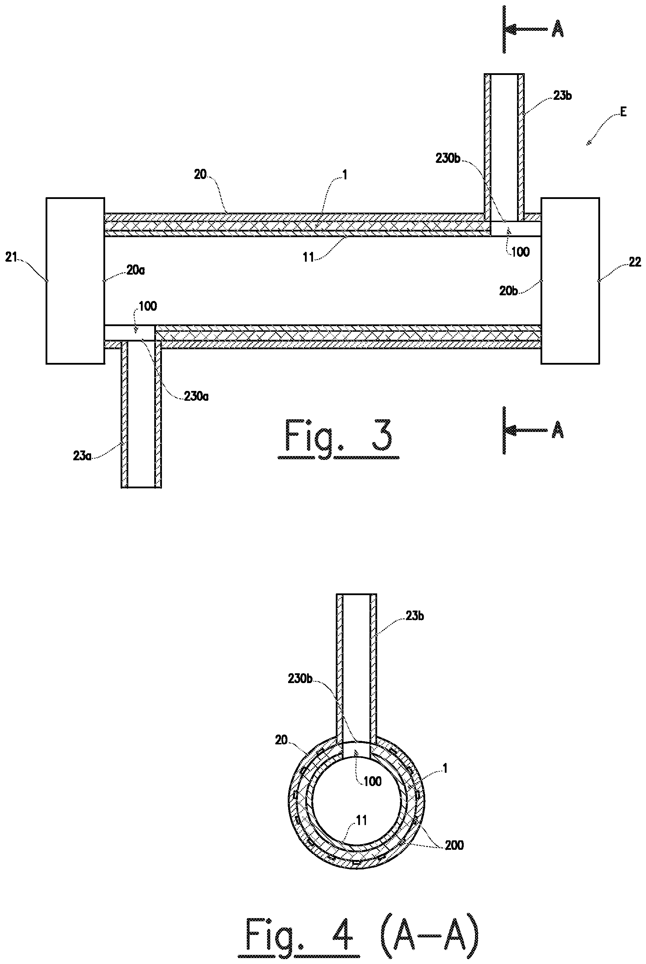

L'intégration du dispositif de chauffage dans la cellule de mesure d'un éthylomètre portable, va maintenant être détaillée en référence aux figures 3 et 4. Cet éthylomètre E est du type décrit dans le document SERES précité. Il comporte une cuve de mesure se présentant sous la forme d'un tube creux 20. Ce dernier est typiquement de section circulaire mais peut être de section carrée, rectangulaire, ovale, etc. Le tube 20 peut être réalisé en métal (ex : aluminium, acier inoxydable, ...) ou en plastique (ex: PVC, ABS). Il peut être obtenu par moulage, par extrusion ou par tout autre procédé convenant à l'homme du métier. Sa surface interne ne nécessite pas de traitement de surface particulier contrairement à celui décrit dans le document SERES.

Selon un mode préféré de réalisation, la longueur du tube 20 est comprise entre 5 mm et 200 mm, préférentiellement inférieure ou égale à 100 mm, l'invention permettant d'utiliser une cuve de mesure plus courte que celle du document SERES. Son diamètre interne est inférieur à 15 mm, par exemple compris entre 4 mm et 15 mm. Et son épaisseur est inférieure à 5 mm, par exemple comprise entre 1 mm et 5 mm.

Une extrémité 20a du tube 20 est pourvue d'un dispositif 21 d'émission d'un rayonnement infrarouge, avantageusement dans des longueurs d'ondes comprises entre 1 µm et 12 µm. L'autre extrémité 20b est pourvue d'un récepteur infrarouge 22. L'émetteur 21 et le récepteur 22 infrarouge sont du type connu de l'homme du métier. Le fluide d'haleine circule dans la cuve de mesure entre les deux extrémités 20a, 20b du tube 20. Plus particulièrement, le fluide pénètre dans le tube 20 par l'intermédiaire d'une tubulure d'entrée 23a (dans laquelle souffle l'utilisateur) installée au niveau de l'extrémité 20a, et ressort dudit tube par l'intermédiaire d'une tubulure de sortie 23b installée au niveau de l'extrémité opposée 23b. Les deux tubulures 23a et 23b peuvent être situées du même côté du tube 20, ou au contraire sur deux côtés opposés (figure 3), ou disposées selon n'importe quel angle. Un système de pompage peut être associé aux tubulures 23a et 23b afin d'assurer la circulation de l'échantillon de fluide soufflé.

Le dispositif de chauffage est inséré dans le tube 20 de manière à ce que le matériau réfléchissant 11 de l'article 1 forme la couche de réflexion optique. Lorsque le tube 20 est de section circulaire, l'article 1 est roulé, manuellement ou automatiquement, de manière à former un cylindre. Dans le cas où le tube 20 n'est pas de section circulaire, mais de section carrée, rectangulaire ou d'une autre forme polygonale, l'article 1 est plié de manière à former un tube ayant cette section particulière. Le matériau réfléchissant 11 forme la surface interne de ce cylindre (ou de ce tube). Cette disposition permet d'optimiser les longueurs des trajets optiques dans le tube 20, tout en conservant une quantité de lumière suffisante jusqu'au récepteur 22. Il en résulte que la cuve de mesure peut être plus courte que celle de l'éthylomètre décrit dans le document SERES.

L'article optique 1 a une longueur correspondante à celle du tube 20 de sorte que la surface interne de ce dernier soit totalement, ou sensiblement totalement, recouverte par ledit article. En effet, certaines zones de la surface interne du tube 20 peuvent ne pas être recouvertes, notamment au niveau des extrémités 20a et 20b, tout en préservant une qualité de mesure acceptable.

L'article 1 ainsi conformé est alors inséré dans le tube 20, au niveau d'une des extrémités 20a ou 20b, de sorte que le matériau réfléchissant 11 forme la couche de réflexion optique contre lequel rebondira le rayonnement infrarouge. Dans la configuration de la figure 1, le support 10 est en contact avec la surface interne du tube 20. Dans la configuration de la figure 2, c'est l'élément chauffant 13 qui est en contact avec la surface interne du tube 20.

Lorsque l'article 1 est mis en forme, il a naturellement tendance à se dérouler (ou se déplier) pour retrouver sa forme plate d'origine. Il résulte de cette aptitude que l'article 1 est naturellement maintenu en position à l'intérieur du tube 20, sans qu'il soit nécessaire de prévoir un autre système de fixation mécanique ou par colle. Un tel système peut toutefois être envisagé par mesure de précaution.

Afin de limiter les déperditions thermiques vers l'extérieur du tube 20, la surface interne de ce dernier comporte avantageusement des éléments en relief 200. Ces derniers consistent par exemple en des nervures longitudinales ou radiales, ou selon toute autre forme présentant des creux et des bosses sur la surface interne du tube 20. Comme cela apparait sur la figure 4, lorsque l'article optique 1 est inséré dans le tube 20, il n'est en contact qu'avec ces éléments en relief. Les ponts thermiques entre le dispositif de chauffage et le tube 1 sont de fait réduits.

Une fois que l'article 1 est mis en forme et installé dans le tube 20, et que la cuve de mesure est ainsi fabriquée, les autres composants 21, 22, 23a, 23b sont mis en place.

Concernant les tubulures 23a, 23b, il est nécessaire qu'elles débouchent à l'intérieur de la cuve de mesure, malgré la présence de l'article 1 qui recouvre la surface interne du tube 20. Pour ce faire, et comme cela apparait sur la figure 5, l'article 1 comprend des encoches, des perçages, ou, de manière plus générale, des évidements 100, dont les dimensions sont ajustées aux diamètres des tubulures 23a, 23b. Ces évidements 100 sont situés au niveau des bords latéraux de l'article 1. Lorsque l'article 1 est mis en forme et inséré dans le tube 10, les évidements 100 sont placés en vis-à-vis des extrémités débouchantes 230a, 230b des tubulures 23a, 23b et laissent libres ces dernières.

L'agencement des différents éléments et/ou moyens et/ou étapes de l'invention, dans les modes de réalisation décrits ci-dessus, ne doit pas être compris comme exigeant un tel agencement dans toutes les implémentations. En tout état de cause, on comprendra que diverses modifications peuvent être apportées à ces éléments et/ou moyens et/ou étapes, sans s'écarter de la portée de l'invention.

## Revendications

1. Appareil de mesure par spectrométrie comprenant une cuve de mesure combinée à un dispositif de chauffage, ladite cuve de mesure se présentant sous la forme d'un tube creux (20), **se caractérisant par le fait que** le dispositif de chauffage se présente sous la forme d'un article optique souple (1), lequel article comprend un support flexible souple (10) présentant une face supérieure (10a) et une face inférieure (10b), la face supérieure (10a) étant recouverte d'un matériau réfléchissant (11) pour former une couche de réflexion optique, un élément chauffant (13) flexible étant disposé sur au moins une desdites faces du support, ledit dispositif de chauffage étant inséré dans ledit tube creux (20) de manière à ce que ledit matériau réfléchissant (11) dudit article optique souple (1) forme ladite couche de réflexion optique.

2. Appareil selon la revendication 1, dans lequel l'élément chauffant (13) est disposé sur la face inférieure (10b) du support flexible (10).

3. Appareil selon la revendication 1, dans lequel l'élément chauffant est disposé sur la face supérieure (10a) du support flexible (10), le matériau réfléchissant (11) recouvrant ledit élément chauffant.

4. Appareil selon l'une des revendications 1 à 3, dans lequel l'élément chauffant (13) se présente sous la forme d'un circuit imprimé flexible dans lequel sont intégrés un ou plusieurs filaments chauffants (130).

5. Appareil selon l'une des revendications 1 à 4, dans lequel l'élément chauffant (13) est associé à une couche (12) d'un matériau conducteur de chaleur.

6. Appareil selon la revendication 5, dans lequel le matériau conducteur de chaleur (12) est choisi dans le groupe suivant : cuivre, aluminium, argent, or.

7. Appareil selon l'une des revendications 1 à 6, dans lequel le support flexible souple (10) est réalisé dans un matériau choisi dans le groupe suivant : polyimide, polyépoxyde, polyester, résine époxy renforcée de fibre de verre, substrat d'aluminium.

8. Appareil selon l'une des revendications 1 à 7, dans lequel le support flexible souple (10) a une épaisseur comprise entre 1 µm et 250 µm.

9. Appareil selon l'une des revendications 1 à 8, dans lequel le matériau réfléchissant (11) formant la couche de réflexion optique, est choisi dans le groupe suivant : or, cobalt, argent, nickel, cuivre, aluminium, chrome, zinc, silice.

10. Ethylomètre pour mesurer ou détecter un taux de gaz partiel exhalé par un fluide d'haleine comprenant un appareil de mesure par spectrométrie selon la revendication 1, ledit éthylomètre comportant un dispositif d'émission (21) d'un rayonnement infrarouge, un récepteur infrarouge (22) et la cuve de mesure dans laquelle peut circuler le fluide d'haleine.

11. Ethylomètre selon la revendication 10, dans lequel le tube creux (20) formant cuve de mesure a une longueur inférieure ou égale à 100 mm, l'article optique (1) ayant une longueur correspondante à celle dudit tube.

12. Ethylomètre selon l'une des revendications 10 ou 11, dans lequel la surface interne du tube creux (20) comporte des éléments en relief (200), l'article optique (1) n'étant en contact avec la surface interne dudit tube qu'au niveau de ces éléments en relief.

13. Procédé de fabrication d'une cuve de mesure d'un éthylomètre permettant de mesurer le taux de gaz partiel exhalé par un fluide d'haleine, laquelle cuve de mesure se présente sous la forme d'un tube creux (20), **se caractérisant par le fait que** le procédé consiste à :
- utiliser un dispositif de chauffage se présentant sous la forme d'un article optique souple (1), lequel article comprend un support flexible souple (10) présentant une face supérieure (10a) et une face inférieure (10b), la face supérieure (10a) étant recouverte d'un matériau réfléchissant (11) pour former une couche de réflexion optique, un élément chauffant (13) flexible étant disposé sur au moins une desdites faces du support,
- rouler ou plier l'article optique (1) du dispositif de chauffage,
- insérer l'article optique (1) ainsi roulé ou plié dans le tube creux (20) de manière à ce que le matériau réfléchissant (11) dudit article forme la couche de réflexion optique.

## Patentansprüche

1. Spektrometriemessvorrichtung, umfassend ein Messgefäß in Kombination mit einer Erwärmungsvorrichtung, wobei das Messgefäß die Form eines hohlen Röhrchens (20) aufweist,
**dadurch gekennzeichnet, dass** die Erwärmungsvorrichtung die Form eines nachgiebigen optischen Artikels (1) aufweist, wobei der Artikel einen nachgiebigen flexiblen Träger (10) mit einer oberen Fläche (10a) und einer unteren Fläche (10b) umfasst, wobei die obere Fläche (10a) mit einem reflektierenden Material (11) bedeckt ist, um eine optische Reflexionsschicht zu bilden, wobei ein flexibles Erwärmungselement (13) auf mindestens einer der Flächen des Trägers angeordnet ist, wobei die Erwärmungsvorrichtung in das hohle Röhrchen (20) derart eingesetzt ist, dass das reflektierende Material (11) des nachgiebigen optischen Artikels (1) die optische Reflexionsschicht bildet.

2. Vorrichtung nach Anspruch 1, wobei das Erwärmungselement (13) auf der unteren Fläche (10b) des flexiblen Trägers (10) angeordnet ist.

3. Vorrichtung nach Anspruch 1, wobei das Erwärmungselement auf der oberen Fläche (10a) des flexiblen Trägers (10) angeordnet ist, wobei das reflektierende Material (11) das Erwärmungselement bedeckt.

4. Vorrichtung nach einem der Ansprüche 1 bis 3, wobei das Erwärmungselement (13) die Form einer flexiblen gedruckten Schaltung aufweist, in der ein oder mehrere Heizfäden (130) integriert sind.

5. Vorrichtung nach einem der Ansprüche 1 bis 4, wobei das Erwärmungselement (13) mit einer Schicht (12) aus einem wärmeleitenden Material assoziiert ist.

6. Vorrichtung nach Anspruch 5, wobei das wärmeleitende Material (12) in der folgenden Gruppe ausgewählt ist: Kupfer, Aluminium, Silber, Gold.

7. Vorrichtung nach einem der Ansprüche 1 bis 6, wobei der nachgiebige flexible Träger (10) aus einem Material ausgewählt ist, das in der folgenden Gruppe ausgewählt ist: Polyimid, Polyepoxid, Polyester, glasfaserverstärktes Epoxyharz, Aluminiumsubstrat.

8. Vorrichtung nach einem der Ansprüche 1 bis 7, wobei der nachgiebige flexible Träger (10) eine Dicke zwischen 1 µm und 250 µm aufweist.

9. Vorrichtung nach einem der Ansprüche 1 bis 8, wobei das reflektierende Material (11), das die optische Reflexionsschicht bildet, in der folgenden Gruppe ausgewählt ist: Gold, Kobalt, Silber, Nickel, Kupfer, Aluminium, Chrom, Zink, Siliciumdioxid.

10. Atemalkoholmessgerät zur Messung oder Detektion eines partiellen Gasgehalts, der von einem Atemfluid ausgeatmet wird, umfassend eine Spektrometriemessvorrichtung nach Anspruch 1, wobei das Atemalkoholmessgerät eine Vorrichtung (21) zur Emission einer Infrarotstrahlung, einen Infrarot-Empfänger (22) und das Messgefäß umfasst, in dem das Atemfluid zirkulieren kann.

11. Atemalkoholmessgerät nach Anspruch 10, wobei das hohe Röhrchen (20), welches das Gefäß bildet, eine Länge kleiner oder gleich 100 mm aufweist, wobei der optische Artikel (1) eine Länge aufweist, die jener des Röhrchens entspricht.

12. Atemalkoholmessgerät nach einem der Ansprüche 10 oder 11, wobei die Innenfläche des hohlen Röhrchens (20) Reliefelemente (200) umfasst, wobei der optische Artikel (1) mit der Innenfläche des Röhrchens nur auf der Höhe dieser Reliefelement in Kontakt steht.

13. Verfahren zur Herstellung eines Messgefäßes eines Atemalkoholmessgeräts, das die Messung des partiellen Gasgehalts ermöglicht, der von einem Atemfluid ausgeatmet wird, wobei das Messgefäß die Form eines hohlen Röhrchens (20) aufweist,
**dadurch gekennzeichnet, dass** das Verfahren besteht aus:
- Verwenden einer Erwärmungsvorrichtung, welche die Form eines nachgiebigen optischen Artikels (1) aufweist, wobei der Artikel einen nachgiebigen flexiblen Träger (10) mit einer oberen Fläche (10a) und einer unteren Fläche (10b) umfasst, wobei die obere Fläche (10a) mit einem reflektierenden Material (11) bedeckt ist, um eine optische Reflexionsschicht zu bilden, wobei ein flexibles Erwärmungselement (13) auf mindestens einer der Flächen des Trägers angeordnet ist,
- Rollen oder Falten des optischen Artikels (1) der Erwärmungsvorrichtung,
- Einsetzen des so gerollten oder gefalteten optischen Artikels (1) in das hohle Röhrchen (20), so dass das reflektierende Material (11) des Artikels die optische Reflexionsschicht bildet.

## Claims

1. Apparatus for measuring by spectrometry, comprising a measuring vessel combined with a heating device, said measuring vessel being in the form of a hollow tube (20), **characterized in that** the heating device is in the form of a soft optical article (1), which article comprises a soft flexible support (10) having an upper face (10a) and a lower face (10b), the upper face (10a) being covered with a reflective material (11) to form an optical reflection layer, a flexible heating element (13) being arranged on at least one of said support faces, said heating device being inserted into said hollow tube (20) such that said reflective material (11) of said soft optical article (1) forms said optical reflection layer.

2. Apparatus according to Claim 1, wherein the heating element (13) is arranged on the lower face (10b) of the flexible support (10).

3. Apparatus according to Claim 1, wherein the heating element is arranged on the upper face (10a) of the flexible support (10), the reflective material (11) covering said heating element.

4. Apparatus according to one of Claims 1 to 3, wherein the heating element (13) is in the form of a flexible printed circuit board into which one or more heating filaments (130) are integrated.

5. Apparatus according to one of Claims 1 to 4, wherein the heating element (13) is associated with a layer (12) of a heat-conducting material.

6. Apparatus according to Claim 5, wherein the heat-conducting material (12) is chosen from the following group: copper, aluminium, silver, gold.

7. Apparatus according to one of Claims 1 to 6, wherein the soft flexible support (10) is made from a material chosen from the following group: polyimide, polyepoxide, polyester, glass fibre-reinforced epoxy resin, aluminium substrate.

8. Apparatus according to one of Claims 1 to 7, wherein the soft flexible support (10) has a thickness of between 1 µm and 250 µm.

9. Apparatus according to one of Claims 1 to 8, wherein the reflective material (11) forming the optical reflection layer is chosen from the following group: gold, cobalt, silver, nickel, copper, aluminium, chromium, zinc, silica.

10. Breathalyser for measuring or detecting a content of constituent gas exhaled in a breath fluid, comprising an apparatus for measuring by spectrometry according to Claim 1, said breathalyser comprising a device for emitting (21) infrared radiation, an infrared receiver (22) and the measuring vessel in which the breath fluid can circulate.

11. Breathalyser according to Claim 10, wherein the hollow tube (20) forming the measuring vessel has a length of less than or equal to 100 mm, the optical article (1) having a length corresponding to that of said tube.

12. Breathalyser according to either of Claims 10 and 11, wherein the inside surface of the hollow tube (20) comprises elements in relief (200), the optical article (1) only being in contact with the inside surface of said tube at these elements in relief.

13. Process for manufacturing a measuring vessel of a breathalyser, making it possible to measure the content of constituent gas exhaled in a breath fluid, which measuring vessel is in the form of a hollow tube (20), **characterized in that** the process consists in:
- using a heating device in the form of a soft optical article (1), which article comprises a soft flexible support (10) having an upper face (10a) and a lower face (10b), the upper face (10a) being covered with a reflective material (11) to form an optical reflection layer, a flexible heating element (13) being arranged on at least one of said support faces,
- rolling or folding the optical article (1) of the heating device,
- inserting the optical article (1) rolled or folded in this way into the hollow tube (20) such that the reflective material (11) of said article forms the optical reflection layer.
